(19) Europäisches Patentamt
European Patent Office
Office européen des brevets

(11) Publication number: **0 177 248**
**B1**

(12) # EUROPEAN PATENT SPECIFICATION

(45) Date of publication of patent specification: **02.09.87**

(51) Int. Cl.$^4$: **C 07 C 113/02**

(21) Application number: **85306773.4**

(22) Date of filing: **24.09.85**

(54) **Process for preparing diazomethane derivative.**

(30) Priority: **27.09.84 JP 203399/84**

(43) Date of publication of application:
**09.04.86 Bulletin 86/15**

(45) Publication of the grant of the patent:
**02.09.87 Bulletin 87/36**

(84) Designated Contracting States:
**BE CH DE GB IT LI**

(56) References cited:

**PATENT ABSTRACTS OF JAPAN, unexamined applications, C section, vol. 3, no. 147, December 5, 1979, THE PATENT OFFICE JAPANESE GOVERNMENT, page 98 C 66**
**PATENT ABSTRACTS OF JAPAN, unexamined applications, C field, vol. 7, no. 143, June 22, 1983, THE PATENT OFFICE JAPANESE GOVERNMENT, page 124 C 172**

(73) Proprietor: **TAOKA CHEMICAL CO., LTD**
**No. 2-11, Nishimikuni 4-chome**
**Yodogawa-ku Osaka-shi Osaka-fu (JP)**

(72) Inventor: **Sakamoto, Sukehiko**
**12-9 Sakurai 4-chome Shimamoto-cho**
**Mishima-gun Osaka-fu (JP)**
Inventor: **Hirayama, Yoshihiko**
**2-9 Mukoyutaka-machi**
**Amagasaki-shi Hyogo-ken (JP)**
Inventor: **Kohno, Yukitomo**
**1-29-102 Mondonishi-machi**
**Nishinomiya-shi Hyogo-ken (JP)**
Inventor: **Sakai, Tamio**
**2-2-6 Sakae-machi**
**Takarazuka-shi Hyogo-ken (JP)**
Inventor: **Shiraishi, Yoshihisa**
**3-c-51-202 Aoyamadai 1-chome**
**Suita-shi Osaka-fu (JP)**
Inventor: **Saijo, Shigeya**
**15-19 Shimoohichi-nishi-machi**
**Nishinomiya-shi Hyogo-ken (JP)**

(74) Representative: **Ford, Michael Frederick et al**
**MEWBURN ELLIS & CO. 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 177 248 B1

Courier Press, Leamington Spa, England.

## Description

Field of the invention

The present invention relates to a process for preparing a diazomethane derivative.

Background of the invention

A diazomethane derivative is known to be useful as a compound providing a protective group for a carboxylic group of an organic compound.

The diazomethane derivative is an easily decomposable compound as apparent from the facts that diazomethane is explosive, diphenyldiazomethane decomposes to form ketazine so that its purity decreases to 75% when it is kept at a room temperature for 2 days (see Organic Synthesis Collective Volume III, page 532).

Therefore, the diazomethane derivatives are stored, transported and used in the form of a solution in a solvent used in the production step, and they have not been crystallized in a commercial scale.

It is commercially disadvantageous to store, transport and handle the diazomethane derivatives in the form of a solution. Further, when the solvent used in the production step is different from that used in the end application of the diazomethane derivative, the solution in the former solvent as such cannot be used in the end application.

As the diazomethane derivatives are used in an increasing amount so that it is highly required to store it for a longer period of time, a safe and general stock form and production manner of the diazomethane derivatives are eagerly desired.

Summary of the invention

One object of the present invention is to provide a process for preparing a stable crystalline diazomethane derivative with high purity in a high yield.

Another object of the present invention is to provide a process for preparing a crystalline diazomethane derivative, all the steps of which may be carried out in one equipment.

Detailed description of the invention

The decomposition properties of crystal and solutions in methylene chloride of 1,1'-diphenyldiazomethane (DDM) have been studied at 0°C to obtain the results as shown in Table 1.

TABLE 1

| Experiment No. | DDM | | Decomposition percentage of DDM | | | |
|---|---|---|---|---|---|---|
| | Conc. (wt.%) | State | 5 days | 10 days | 20 days | 40 days |
| 1 | 20 | Solution | 17.1 | 19.0 | 29.0 | 50.2 |
| 2 | 30 | Solution | 3.0 | 5.6 | 15.8 | 48.0 |
| 3 | 50 | Solution | 1.5 | 2.0 | 4.9 | 30.5 |
| 4 | 70 | Slurry | 0.9 | 1.8 | 3.4 | 14.2 |
| 5 | 90 | Slurry | 0.2 | 0.2 | 0.4 | 1.2 |
| 6 | 100 | Crystal | 0.1 | 0.2 | 0.3 | 1.1 |

According to the results, as the concentration of DDM in the solution increases, the stability increases. The slurry which contains crystal of DDM is more stable than the solution and the crystalline DDM which contains no solvent is most stable.

Decomposability of DDM due to heat history and stability against friction or impact also have been studied.

With the use of DDM having purity of 99.9%, decomposability at various temperatures has been examined. The results are shown in Table 2.

TABLE 2

| Storage temperature (°C) | Decomposition percentage of DDM | | | | |
|---|---|---|---|---|---|
| | 2 hrs. | 10 hrs. | 20 hrs. | 50 hrs. | 100 hrs. |
| −25 | 0 | 0 | 0 | 0 | 0 |
| 0 | 0 | 0 | 0 | 0 | 0 |
| 10 | 0.1 | 0.1 | 0.1 | 0.2 | 0.2 |
| 20 | 0.2 | 0.6 | 0.7 | 1.0 | 2.5 |
| 25 | 0.4 | 0.9 | 1.8 | 4.4 | 8.6 |
| 30 (melt) | 1.4 | 6.8 | 13.2 | 29.8 | 50.7 |
| 40 (melt) | 4.0 | 17.7 | 32.2 | 62.2 | 85.7 |
| 50 (melt) | 5.5 | 39.0 | 90.5 | 100 | 100 |

As understood from the results of Table 2, DDM does not substantially decompose at a temperature lower than 5°C, but gradually starts to decompose at a temperature higher than 10°C, and about 5% decomposes at 25°C after 50 hours and 5% or more at 30°C after only several hours. Since the melting point of DDM is about 29—31°C, it melts at a temperature higher than 29°C and it quickly decomposes in a melt state. This is further confirmed by the following experiments.

DDM with purity of 99.9% is once molten at 30°C and kept in a supercooling state while maintaining the melt state at various temperatures. The decomposition percentages are shown in Table 3.

TABLE 3

| Storage conditions | | Decomposition percentage | | | | |
|---|---|---|---|---|---|---|
| Temp. | State | 2 hrs. | 10 hrs. | 20 hrs. | 50 hrs. | 100 hrs. |
| 5°C | Crystal | 0 | 0 | 0 | 0 | 0 |
| | Melt | 0.3 | 1.3 | 2.5 | 6.2 | 12.0 |
| 15°C | Crystal | 0.1 | 0.1 | 0.2 | 0.4 | 0.6 |
| | Melt | 0.3 | 1.4 | 2.6 | 6.5 | 13.1 |
| 25°C | Crystal | 0.4 | 0.9 | 1.8 | 4.4 | 8.6 |
| | Melt | 0.9 | 4.3 | 8.3 | 19.5 | 35.4 |

As is apparent from the results of Table 3, the decomposition rate in the melt state is far greater than that in the crystalline state at the same temperature. From the analysis of these data according to reaction kinetics, it has been found that the decomposition reaction is a first order reaction and its activation energies in the crystalline and melt states are 72.7 kcal/mol and 19.4 kcal/mol, respectively. Since an activation energy of a very easily decomposable organic peroxide is about 20—30 kcal/mol, DDM in the melt state decomposes as easily as or more easily than the organic peroxide.

To examine the stability of DDM against friction and impact, the same pure DDM as used in the above experiment (purity 99.9%) was subjected to drop hammer test, friction test (according to JIS K 4810—1797), ignition test (according to the Crupp method), steel pipe test, pressure container test (cf. D. C. Noelor et al: Ind. Eng. Chem. 5618 (1964)). The results are as follows:

| | |
|---|---|
| Drop hammer test: | 8th class |
| Friction test: | 7th class |
| Ignition point: | 136—139°C |
| Steel pipe test: | 6th class |
| Pressure container test: | burst with No. 7 orifice |

From all the above results, it is concluded that DDM is comparatively stable against friction and impact so that it does not quickly decompose and explode, it gradually decomposes as heated but quickly decomposes in the melt state. Further, the solution of DDM becomes very viscous as it is crystallized and dried so that it easily adheres on an equipment for treatment and prevents heat transfer or decreases drying efficiency, which results in low yield.

Thus, the present invention relates to a stable crystalline diazomethane derivative and a safe process for preparing a diazomethane derivative. By the crystallization of the diazomethane derivatives, its application fields will be broadened.

Accordingly, the present invention provides a process for preparing a crystalline diazomethane derivative of the formula:

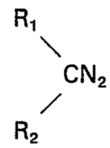

(I)

wherein $R_1$ and $R_2$ are, the same or different, a substituted or unsubstituted aromatic group, which comprises concentrating and drying a solution of the diazomethane derivative in an organic solvent under reduced pressure at a temperature not higher than 30°C while applying shearing force on a viscous cake.

Specific examples of the diazomethane derivative (I) are diphenyldiazomethane, p-methyldiphenyldiazomethane, p-chlorodiphenyldiazomethane, p-nitrodiphenyldiazomethane, p,p'-dichlorodiphenyldiazomethane and the like.

Specific examples of the organic solvent are methylene chloride, 1,2-dichloroethane, chloroform, ethyl acetate, ethyl ether, petroleum ether, n-hexane, n-heptane, benzene, and the like. The solution to be treated by the process of the present invention may be a solution obtained in the production step of the diazomethane derivative in the above solvent or a solution made by dissolving the crystalline diazomethane derivative in the above solvent. Since it is preferable to carry out the concentration and drying of the solution as quickly as possible for effective preparation of the diazomethane derivative according to the present invention, the solvent has as low boiling point as possible and no flammability. Among the solvents, methylene chloride is most preferred.

The diazomethane derivative (I) may be prepared by a per se conventional method, for example, comprising oxidizing a corresponding hydrazone derivative with a suitable oxidizing agent (e.g., manganese dioxide, mercury oxide, silver oxide, peracetic acid, hydrogen peroxide, etc.). Particularly, electrolytic manganese dioxide is preferred since it gives the highly pure diazomethane derivative in a high yield.

The concentration and drying of the solution of the diazomethane derivative are carried out under reduced pressure at a temperature not higher than 30°C, preferably not higher than 25°C and not lower than −25°C. At a temperature higher than 30°C, the crystalline diazomethane derivative melts and quickly decomposes. This leads to the deterioration of the quality and yield of the product. The pressure depends on other conditions such as the kind of the solvent, the concentration of the solution, the temperature, etc.

As the concentration and drying of the solution proceed, the solution becomes a very viscous cake and tends to adhere on a stirrer or other equipments. However, when the viscous cake of the diazomethane derivative is further dried with applying the shearing force thereon, the cake gradually loses adhesivity and finally powdered.

Although the present process can be carried out in one step, the solution may be concentrated to form a slurry in one equipment and then dried by means of a dryer which can apply the shearing force.

Examples of an equipment which can apply the shearing force on its content are a dryer with a self-contained vertical type screw or ribbon, a vacuum rotary dryer, a Uninix dryer, and the like. Among them, the most preferred is the dryer with the self-contained vertical type screw or ribbon, for example, a Nauta type dryer, since only a small amount of the cake adheres to this type of dryer and the yield of the powder is increased.

The present invention will be hereinafter explained further in detail by following examples.

Example 1

A 50% solution of diphenyldiazomethane (20 kg, purity 99.0%) in methylene chloride (20 kg) was charged in a Nauta type 50 l dryer (a vacuum dryer consisting of a reverse cone shaped vessel equipped with a rotating and orbiting screw type agitator and a jacket. Rotational speed 70 RPM. Orbiting speed: 2.6 RPM). The internal pressure was gradually reduced to 10 Torr while keeping the jacket temperature between −7 and −2°C to obtain diphenyldiazomethane (19 kg) with purity of 98.9%.

Example 2

A 50% solution of diphenyldiazomethane (2 kg, purity 99.0%) in methylene chloride (2 kg) was charged in a 10 l vacuum rotary dryer (consisting of an agitator having four lines of blades (on each line, 5 flat blades are obliquely attached) and a jacket. Rotational speed: 8 RPM). The internal pressure was gradually reduced to 10 Torr while keeping the jacket temperature between −5 and 0°C to obtain diphenyldiazomethane (1.6 kg) with purity of 98.8%.

Example 3

To a glass-lined 100 l reactor, methylene chloride (22 kg) was charged and benzophenone hydrazone (29 kg) was mixed. To the mixture, electrolytic manganese dioxide containing 91.0% of effective oxygen (39 kg) was added at a temperature of 20 to 25°C over 2 hours and then stirred at the same temperature for 2 hours. Then, the reaction mixture was filtered and the filter cake was washed with methylene chloride. The washing solvent was combined with the filtrate. The purity of the product in the combined solution was 30%. The solution was charged in the same Nauta type dryer as used in Example 1. The internal pressure was gradually reduced to 10 Torr while keeping the jacket temperature between 10 and 15°C (the internal temperature 5 to 15°C) to obtain diphenyldiazomethane (26 kg) having purity of 99.0%. Yield 91%.

**Claims**

1. A process for preparing a crystalline diazomethane derivative of the formula:

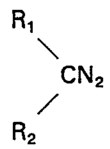

(I)

wherein $R_1$ and $R_2$ are, the same or different, a substituted or unsubstituted aromatic group, which comprises concentrating and drying a solution of the diazomethane derivative in an organic solvent under reduced pressure at a temperature not higher than 30°C while applying shearing force on a viscous cake.

2. A process according to claim 1, wherein the organic solvent is one selected from the group consisting of methylene chloride, 1,2-dichloroethane, chloroform, ethyl acetate, ethyl ether, petroleum ether, n-hexane, n-heptane and benzene.

3. A process according to claim 2, wherein the organic solvent is methylene chloride.

4. A process according to any one of the preceding claims, wherein the temperature is from −25 to +25°C.

5. A process for preparing a crystalline diazomethane derivative of the formula:

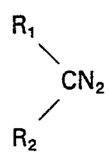

(I)

wherein $R_1$ and $R_2$ are, the same or different, a substituted or unsubstituted aromatic group, which comprises concentrating and drying a solution of the diazomethane derivative in an organic solvent under sub-atmospheric pressure at a temperature not higher than 30°C until a cake of partially solidified derivative forms, and thereafter continuing concentration and drying of the cake under sub-atmospheric pressure at a temperature not higher than 30°C while applying shearing force to the cake.

**Patentansprüche**

1. Verfahren zur Herstellung eines kristallinen Diazomethanderivats der Formel·

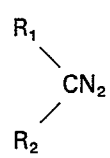

(I)

in der $R_1$ und $R_2$ gleich oder verschieden sind und eine substituierte oder unsubstituierte aromatische Gruppe bedeuten, durch Einengen und Trocknen einer Lösung des Diazomethanderivats in einem organischen Lösungsmittel unter vermindertem Druck bei einer Temperatur nicht höher als 30°C unter Ausübung einer Scherkraft auf den viskosen Kuchen.

2. Verfahren nach Anspruch 1, wobei das organische Lösungsmittel Methylenchlorid, 1,2-Dichloräthan, Chloroform, Äthylacetat, Äthyläther, Petroläther, n-Hexan, n-Heptan oder Benzol ist.

3. Verfahren nach Anspruch 2, wobei das organische Lösungsmittel Methylenchlorid ist.

4. Verfahren nach einem der vorstehenden Ansprüche, wobei die Temperatur −25 bis +25°C beträgt.

5. Verfahren zur Herstellung eines kristallinen Diazomethanderivats der Formel

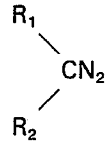

(I)

in der $R_1$ und $R_2$ gleich oder verschieden sind und eine substituierte oder unsubstituierte aromatische Gruppe bedeuten, durch Einengen und Trocknen einer Lösung des Diazomethanderivats in einem organischen Lösungsmittels unter subatmosphärischem Druck bei einer Temperatur nicht über 30°C bis ein Kuchen des teilweise verfestigten Derivates entstanden ist und anschließend weiteres Einengen und Trocknen des Kuchens unter subatmosphärischem Druck bei einer Temperatur nicht über 30°C unter Ausübung einer Scherkraft auf den Kuchen.

**Revendications**

1. Procédé de préparation d'un dérivé cristallisé du diazométhane de la formule

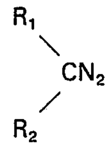

(I),

dans laquelle $R_1$ et $R_2$, qui peuvent être identiques ou différentes, désignent chacun un groupe aromatique substitué ou non substitué, qui consiste à concentrer et à sécher une solution du dérivé du diazométhane dans un solvant organique, sous une pression réduite et à une température ne dépassant pas 30°C et en soumettant le gâteau visqueux à une force de cisaillement.

2. Procédé suivant la revendication 1, dans lequel le solvant organique est choisi parmi le chlorure de méthylène, le 1,2-dichlor-éthane, le chloroforme, l'acétate d'éthyle, l'éther éthylique, l'éther de pétrole, le n-hexane, le n-heptane et le benzène.

3. Procédé suivant la revendication 2, dans lequel le solvant organique est le chlorure de méthylène.

4. Procédé suivant l'une quelconque des revendications précédentes, dans lequel la température est maintenue entre −25 et +25°C.

5. Procédé de préparation d'un dérivé cristallisé du diazométhane de la formule

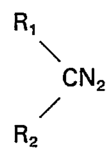

(I),

dans laquelle $R_1$ et $R_2$, qui peuvent être identiques ou différents, désignent chacun un groupe aromatique substitué ou non substitué, qui consiste à concentrer et à sécher une solution du dérivé du diazométhane dans un solvant organique à une pression inférieure à la pression atmosphérique et à une température ne dépassant pas 30°C jusqu'à la formation d'un gâteau du dérivé partiellement solidifié, puis à poursuivre la concentration et le séchage à une pression inférieure à la pression atmosphérique et à une température ne dépassant pas 30°C en soumettant le gâteau à une force de cisaillement.